# FASCICULE DE BREVET EUROPEEN

(11) **EP 1 239 022 B1**
(45) Date de publication et mention de la délivrance du brevet: **06.05.2009**
(21) Numéro de dépôt: 01105960.7
(22) Date de dépôt: 09.03.2001
(51) Int. Cl.: C11B 1/10, A23L 1/30, A23K 1/16, A23D 9/00, A23C 11/04, C12P 7/64, A61K 8/37

(54) **Huile contenant un ou des acide(s) gras polyinsaturé(s) à longue chaîne issus de biomasse, procédé de Préparation, allment, composition nutritionnelle, cosmétique ou pharmaceutique la contenant**
Öl, das langkettige, mehrfach ungesättigte Fettsäuren aus Biomassen enthält, Verfahren zur Herstellung, Lebensmittel, Nahrungsmittelzusammensetzung, kosmetische oder pharmazeutische Zusammensetzung, die dieses enthält
Oil containing one or more long chain polyunsaturated fatty acids from biomass, process for preparing, food, nutritional composition, cosmetic or pharmaceutical composition containing the same

(43) Date de publication de la demande: 11.09.2002
(73) Titulaire: SOCIETE DES PRODUITS NESTLE S.A., 1800 Vevey (CH)
(72) Inventeur: Bertholet, Raymond, 1807 Blonay (CH); Wang, Junkuan, 1010 Lausanne (CH); Lambelet, Pierre, 1806 Saint-Legier (CH); Watzke, Heribert, 1009 Pully (CH); Kratky, Zdenek, 1613 Maracon (CH)
(74) Mandataire: Dixon, Sarah

(56) Documents cités:
- EP-A- 0 612 725
- WO-A-97/37032
- DATABASE WPI Section Ch, Week 198426 Derwent Publications Ltd., London, GB; Class C03, AN 1984-164291 XP002175534 & SU 1 049 431 A (PLANT PHYSIOL INST), 23 octobre 1983 (1983-10-23)
- MING H. CHENG ET AL.: "Fungal production of eicosapentaenoic and arachidonic acids from industrial waste streams and crude soybean oil" BIORESOURCE TECHNOLOGY., vol. 67, no. 2, 1999, pages 101-110, XP001016139 ELSEVIER., GB ISSN: 0960-8524
- DATABASE WPI Section Ch, Week 199206 Derwent Publications Ltd., London, GB; Class D13, AN 1992-046795 XP002175535 & SU 1 648 322 A (MOSC BR MASLOZHIRPR), 15 mai 1991 (1991-05-15)

## Description

### Introduction

La présente invention a trait au domaine de la préparation d'une huile servant d'ingrédient source d'acides gras essentiels polyinsaturés à longue chaîne (LC-PUFA) dans un aliment, dans un supplément nutritionnel, dans une composition cosmétique ou pharmaceutique.

### Etat de la technique

Une huile contenant des LC-PUFA comme par exemple les acides arachidonique (ARA), docosahexaénoique (DHA) ou dihomogammalinolénique (DHGLA) peut être obtenue à partir d'un bouillon de fermentation d'une biomasse. Pour obtenir l'huile de la biomasse on a utilisé des méthodes d'extraction par solvant organique, par exemple l'hexane ou par fluide supercritique. Généralement, l'huile a été extraite à partir de biomasse par percolation à l'hexane de la biomasse séchée.

Un tel procédé d'extraction par solvant(s) organique(s) est décrit par exemple dans WO9737032, dans WO 9743362 ou dans la publication Journal of Dispersion Science and Technology, 10, 561-579, 1989 "Biotechnological processes for the production of PUFAs".

Cette technique présente différents inconvénients:
- Lors des étapes d'extraction par solvant chaud et de distillation du solvant les LC-PUFA peuvent subir une dégradation au contact de l'oxygène.
- L'élimination totale du solvant contenu dans l'huile ou dans la biomasse résiduel exige un traitement thermique à température élevée.
- Par ailleurs, le solvant, tel que l'hexane est susceptible de dissoudre des constituants non-triacylglycérols de la biomasse qui constituent en fait des impuretés.

L'huile brute obtenue après évaporation du solvant doit encore subir plusieurs étapes de raffinage comprenant un dégommage, une neutralisation à l'alcali, une décoloration , un décirage et une désodorisation dans le but d'éliminer au moins partiellement les impuretés. Cela implique que l'huile très insaturée est exposée à des conditions stimulant des réactions physico-chimiques qui affectent sa qualité. Par exemple, les agents de décoloration créent un système de doubles liaisons conjuguées et forment des produits de dégradation par réaction chimique avec les glycérides oxydés.

EP 612 725 décrit un procédé d'extraction de β-carotene provenant d'une biomasse dans lequel on mélange une huile végétale avec une suspension de la biomasse dans l'eau et on sépare l'huile végétale contenant des β-carotene par un procédé d'ultrafiltration ou microfiltration.

La présente invention a pour but d'éviter les inconvénients de l'art antérieur, en proposant une huile stable contenant un ou des acides gras polyinsaturés issus de biomasse sous forme de triacylglycérols à l'état purifié et ayant subi un minimum de dégradation.

### Résumé de l'invention

La présente invention concerne une huile stable contenant des LC-PUFA sous forme de triacylglycérols, notamment les acides arachidonique (ARA), dihomogammalinolénique (DHGLA), docosahexaénoique (DHA), ou eicosapentaenoique (EPA).

L'invention concerne également un procédé de préparation d'une telle huile selon la revendication 5.

L'huile ne contient pas plus de 10 % en poids d'acides gras polyinsaturés à longue chaîne. De ce fait, l'huile est beaucoup moins sensible à l'oxydation lors de sa production ce qui n'est pas le cas pour les huiles contenant des LC-PUFA de l'art antérieur.

Selon un aspect principal de l'invention, c'est un avantage qualitatif déterminant de disposer d'une huile nouvelle, contenant des LC-PUFA sous forme de triacylglycérols.

### Description détaillée de l'invention

La transformation est réalisée par mise en contact de l'huile support avec une biomasse contenant des LC-PUFA. L'huile est adaptée à l'application dans les aliments, en particulier les formules infantiles ou à l'utilisation comme supplément nutritionnel. Elle peut également être utilisée dans des produits cosmétiques ou pharmaceutiques. De plus, le résidu de biomasse obtenu est également un produit du procédé qui peut être valorisé directement sans traitement ultérieur, par exemple comme aliment pour animaux, en particulier pour animaux familiers.

La préparation d'une telle huile peut avoir lieu par simple mélange de l'huile support avec la biomasse séchée et séparation ultérieure de l'huile d'avec les solides non lipidiques par pressage.

En vue d'augmenter le rendement en LC-PUFA obtenu, il est préférable de réduire les dimensions des particules de biomasse sèche pour briser les parois des cellules de micoorganismes et augmenter ainsi la surface de contact entre l'huile et la biomasse. Cela peut être réalisé de manière appropriée par utilisation de différentes méthodes, par exemple:
- on peut broyer la biomasse en présence de l'huile support ;
- on peut laminer la biomasse avant de la mélanger avec l'huile support;
- on peut traiter la biomasse sous haute pression en présence de l'huile support ; puis séparer l'huile obtenue d'avec la biomasse par pressage et filtration de finition.
- on peut traiter la biomasse avec des enzymes capables de dégrader les parois des cellules.

Du fait que le support est une huile, l'huile obtenue après contact avec la biomasse a un contenu minimum en phospholipides, acides gras libres, pigments, polymères et autres substances issues ou dérivées de la biomasse qui ne sont pas des triacylglycérols. Cela signifie que le procédé selon l'invention constitue une méthode sélective de préparation d'une huile purifiée stable contenant des LC-PUFA. Il n'est pas nécessaire de purifier l'huile insaturée contenant les LC-PUFA par les méthodes agressives et lourdes utilisées antérieurement à l'invention que sont les étapes de dégommage, neutralisation, décirage et décoloration.

Selon l'invention, l'huile est soumise uniquement à une étape de désodorisation, par exemple par entraînement à la vapeur ou distillation moléculaire à température relativement basse. Il en résulte que l'huile contient une quantité particulièrement faible d'acides gras trans.

Le procédé n'utilise pas de solvant organique et, dans la mesure où l'on travaille sous couche d'azote et en présence de tocophérols naturellement présents ou rajoutés dans l'huile support, les LC-PUFA sont protégés de la dégradation oxydative pendant tout le procédé.

En plus de la qualité de l'huile obtenue, un autre avantage du procédé consiste dans le fait que le résidu de biomasse n'est pas contaminé par un solvant organique et peut ainsi être valorisé directement, sans traitement ultérieur, par exemple dans des aliments pour animaux, notamment pour animaux familiers.

La description détaillée du procédé qui suit est ciblée sur la préparation d'une huile contenant l'ARA, pris à titre d'exemple, non limitatif. Les conditions de travail pour transférer d'autres LC-PUFA vers une huile support à partir des biomasses appropriées, par exemple pour le DHA ou le DHGLA sont très proches.

L'huile est obtenue par mélange de l'huile support avec la biomasse sèche et séparation de l'huile d'avec les composants solides par pressage. Pour augmenter le taux d'incorporation en ARA, il est souhaitable de rompre les cellules microbiennes par des traitements sous haute pression, par des procédés enzymatiques ou de réduire les dimensions des particules sèches de biomasse par mouture ou laminage.

L'étape de mouture mise en oeuvre peut être l'une des nombreuses techniques connues de l'art antérieur. Par exemple, on peut laminer la biomasse, de préférence à basse température, puis on peut la mélanger avec l'huile support. En variante, on peut moudre la biomasse en présence de l'huile support. En vue de minimiser autant que possible la détérioration de l'ARA, les conditions de mouture doivent être douces. Dans cette optique, on préfère moudre la biomasse en présence de l'huile support et sous atmosphère inerte, par exemple sous balayage d'azote.

Ensuite, on sépare l'huile contenant l'ARA d'avec la biomasse par filtration ou pressage, de préférence sous forte pression, puis on effectue une filtration de finition de manière à éliminer les particules fines de biomasse.

On a constaté que le taux d'incorporation en ARA augmentait lorsque la taille des particules de biomasse diminuait, celui-ci était > 90% lorsque par exemple 90% des particules avaient une dimension < 250 µm.

A titre d'exemple, on peut utiliser un moulin à billes ou un moulin colloïdal. Les paramètres à considérer sont la durée de mouture, la taille des particules de biomasse, la température de mouture, le rapport entre les quantités de biomasse et d'huile support.

La durée de mouture a une influence sur la taille des particules et cette dernière est également influencée par la température de mouture. Par suite, en pratique, on préfère indiquer la taille des particules comme paramètre déterminant de l'étape de mouture. Ainsi, il est souhaitable que 90 % des particules soient de dimension < 500 µm, de préférence que 90 % des particules soient de dimension < 300 µm et de manière encore plus préférée que 90 % des particules soient de dimension< 200 µm.

La température de mouture est choisie à une valeur supérieure au point de fusion de l'huile support, et est de préférence de 20 à 80° C. Pour obtenir un taux d'incorporation optimal, on peut effectuer une mouture brève à température élevée ou une mouture prolongée à basse température.

Le rapport pondéral choisi entre la biomasse et l'huile support détermine le contenu en ARA de l'huile finale. Ainsi, par exemple, on choisit 30 parties de biomasse pour 70 parties d'huile support pour obtenir au moins 4,5 % d'ARA dans l'huile transformée.

L'huile utilisée comme support peut être n'importe quelle huile ou mélange d'huiles consommable en alimentation humaine. On utilise de préférence une huile ou un mélange entrant dans la composition du produit que l'on souhaite enrichir en PUFA. On peut citer en particulier pour une formule infantile l'huile de tournesol riche en acide oléique (HOSFO), l'huile de tournesol (SFO), l'huile de soja, l'oléine de palme et un triacyglycérol à chaîne moyenne (MCT, contenant essentiellement des triacylglycérols d'acides gras saturés en C₈-C₁₀).

L'étape suivante du procédé consiste à séparer le résidu de biomasse épuisée par toute méthode usuelle comme par exemple le pressage, la filtration ou la centrifugation. A cet effet on utilise de préférence une presse opérant sous forte pression.

L'huile obtenue doit être débarrassée des particules fines insolubles par filtration fine. Cette opération peut être effectuée le cas échéant en mettant l'huile en présence d'un adsorbant minéral en qualité d'aide à la filtration, par exemple la dicalite.

Enfin, l'huile filtrée est désodorisée pour en éliminer les substances volatiles. Cela peut être réalisé par toute méthode connue pourvu que cela soit dans des conditions modérées de manière à ménager l'ARA. On peut citer, par exemple, l'entraînement à la vapeur, de préférence sous vide ou la distillation moléculaire.

L'huile obtenue peut être utilisée dans des compositions alimentaires pour l'alimentation humaine telle quelle ou sous forme d'émulsion comme par exemple les huiles, sauces pour salade ou mayonnaises. Elle peut être un constituant d'un lait diététique pour adolescents ou adultes, d'une formule infantile pour prématurés, nourrissons nés à terme ou un lait de suite pour petits enfants.

Elle peut être incorporée dans une composition nutritive ou de supplémentation, pour l'alimenation orale.

Elle peut être incorporée dans une composition pharmaceutique pour l'ingestion orale, entérale ou parentérale, ou pour l'application topique dermatologique ou ophtalmique.

Elle peut constituer un ingrédient d'une composition cosmétique, topique ou orale.

Enfin, elle peut constituer un ingrédient d'un aliment pour animaux familiers, par exemple un aliment sec, humide ou un lait.

Le résidu de biomasse après séparation de l'huile peut avantageusement être utilisé dans les aliments pour animaux, particulièrement les animaux familiers.

### Exemples

Les exemples ci-après illustrent l'invention. Dans ceux-ci, les parties et pourcentages sont pondéraux, sauf indication contraire.
Le rapport biomasse:huile support est de 3:7.

### Exemples 1-6

Dans ces exemples, on étudie les paramètres du procédé et la qualité de l'huile obtenue, avant l'étape finale de désodorisation, en comparaison avec l'huile support de départ (référence 1). Pour la mouture, on utilise un moulin à billes. Les résultats sont résumés dans le tableau 1 ci-après:

**Tableau 1**

| Exemple | 1 | 2 | 3 | 4 | 5 | 6 | Référence 1 |
|---|---|---|---|---|---|---|---|
| Température de mouture (°C) | 50 | 70 | 50 | 30 | 70 | 30 | Huile support* |
| Durée de mouture (min) | 3 | 5 | 3 | 5 | 1 | 1 | - |
| Acides gras libres (%) IUPAC 2.201 | 0,13 | 0,13 | 0,14 | 0,12 | 0,17 | 0,15 | 0,14 |
| Indice de peroxide (meq/kg) AOCS Cd 8b-90 | 4,0 | 4,1 | 3,5 | 3,5 | 4,0 | 3,4 | 2,7 |
| Insaponifiables (g/kg) IUPAC 2.401 | 8,34 | 9,20 | 8,82 | 8,03 | 8,11 | 7,44 | 6,78 |
| ARA (g ARA/100g huile) IUPAC 2.304 | 4,4 | 4,87 | 4,25 | 4,46 | 3,81 | 3,27 | 0 |
| Phosphore (ppm) NI C12-1976-SSOG | 2 | 1 | 3 | 2 | 2 | 4 | 6 |

| | | | | | | | |
|---|---|---|---|---|---|---|---|
| * L'huile support est une huile de tournesol riche en acide oléique, TRISUN ™. | | | | | | | |

La valeur théorique d'incorporation de 100 % d'ARA est 5,3 % avec un rapport biomasse:huile support de 3:7.

La pureté de l'huile brute peut être qualifiée par les valeurs ci-après:
- Acides gras libres: 0,13-0,17 % (TRISUN, huile support: 0,14 %)
- Phosphore: 1-4 ppm (partie par million) (TRISUN: 6 ppm)
- Matière insaponifiable: 7,4-9,2 g/kg (TRISUN: 6,8 g/kg)

En conclusion:
- Plus de 60 % de l'ARA de la biomasse est incorporé dans l'huile support.
- Le contenu en phosphore est très bas, quelques ppm, environ 100 x moins que dans le cas de l'huile brute extraite à l'hexane qui était d'environ 500 ppm.

### Exemples 7-10

Dans ces exemples, on étudie les paramètres du procédé et la qualité de l'huile obtenue, après l'étape finale de désodorisation, lors d'utilisation de différentes huiles supports. Dans ces exemples, la mouture de la biomasse se fait à l'aide d'un moulin à billes.
On compare les caractéristiques des huiles obtenues, en comparaison avec l'huile brute obtenue par extraction à l'hexane, sans raffinage (référence 2) et en comparaison avec l'huile obtenue par pressage direct, donc sans huile support (référence 3).
Les résultats sont résumés dans le tableau 2 ci-après:

**Tableau 2**

| Exemple | 7 | 8 | 9 | 10 | Référence 2 | Référence 3 |
|---|---|---|---|---|---|---|
| Huile support | HOSFO* | HOSFO* | MCT | Oléine de palme | | |
| Conditions de mouture: température (°C), | 70 | 30 | 70 | 70 | | |
| Durée (min) | 5 | 10 | 5 | 5 | | |
| Acides gras libres (%) | 0,04 | 0,03 | 0,04 | 0,04 | 0,56 | 0,11 |
| Indice de peroxyde (meq/kg) | 2,0 | 2,3 | 2,3 | 1,3 | 11,5 | 4,8 |
| Matière insaponifiable (g/kg) | 8,02 | 7,05 | 3,53 | 5,40 | 22,89 | 17,59 |
| Phosphore (ppm) | 3 | 4 | 4 | 3 | 508 | 17 |
| ARA(g ARA/100g huile) | 4,6 | 4,7 | 4,5 | 4,4 | 39,5 | 39,6 |

| | | | | | | |
|---|---|---|---|---|---|---|
| *L'huile support est une huile de tournesol riche en acide oléique (HOSFO). | | | | | | |

Les résultats obtenus permettent les conclusions suivantes:
- La température et la durée de mouture sont couplées: une mouture de 10 min à 30 °C donne le même taux d'incorporation en ARA qu'une mouture pendant 5 min à 70 °C
- Le taux d'incorporation dépend seulement légèrement du type d'huile support lorsque l'on opère à la même température/durée de mouture: huile de tournesol riche en acide oléique (4,9 % ARA à 70 °C/5min), MCT (5,0 % ARA à
   70 °C/5min) et oléine de palme (5,0 % ARA à 70 °C/5min).
- On obtient une très faible quantité de phosphore par rapport à celle obtenue par extraction à l'hexane, ce qui montre la pureté de l'huile finale.

### Exemples 11-14

Les exemples ci-après montrent la préparation d'une huile contenant l'ARA sous forme de triacylglycérols par un procédé ménageant la qualité de l'ARA en utilisant plusieurs voies, sans mouture (exemple 11) et avec différents équipements de mouture (exemples 12-14).

### Matières utilisées:

Biomasse contenant 36,3 % d'huile à 39,5 % d'acide arachidonique (ARA). Huile de tournesol riche en acide oléique.
Oléine de palme.
Huile MCT.

### Exemple 11- Préparation par contactage avec de l'huile de tournesol riche en acide oléique.

### Equipement:

Réacteur agité en verre de 1000 ml avec double-manteau, relié à un bain thermostaté.
Presse Carver avec cartouche filtrante 48 x 200 mm.
Filtre-cloche thermostaté MAVAG 300 ml.
Désodoriseur de laboratoire selon J.HEIDE-JENSEN (JAOCS; Vol. 40; 223-224; 1963) avec ballon de 1000 ml.

### Procédure:

On introduit 260 g d'huile de tournesol riche en acide oléique et 112 g de biomasse dans le réacteur. On met le récipient sous vide et on remplace l'air par de l'azote à trois reprises pour l'inertisation. On agite ensuite le réacteur à 50 °C pendant 2 h, puis on récupère le mélange dans une cartouche filtrante. On sépare l'huile de la biomasse par pressage. On recueille 260 g d'huile et 110 g de tourteau.
On filtre l'huile pressée à 50 °C, puis on la désodorise à 180 °C, 1 mbar pendant 2,6 h. On obtient finalement 240 g d'une huile limpide d'odeur neutre et de coloration jaune-claire. On détermine la teneur en ARA de l'huile par l'analyse chromatographique en phase gazeuse (GC) et on calcule le taux d'incorporation de l'ARA.

### Exemple 12.- Préparation par broyage avec de l'oléine de palme dans un broyeur à billes.

### Equipement:

Broyeur à billes DYNO-MILL Type KDL avec récipient de broyage 0,3 1, double-manteau relié à un bain thermostaté.
Presse Carver avec cartouche filtrante 48 x 200 mm.
Filtre-cloche thermostaté MAVAG 300 ml.
Désodoriseur de laboratoire selon J.HEIDE-JENSEN (JAOCS; Vol. 40; 223-224; 1963) avec ballon de 1000 ml.

### Procédure:

On introduit 130 g d'oléine de palme et 56 g de biomasse dans le récipient du broyeur. On met le récipient sous vide et on remplace l'air par de l'azote à trois reprises pour l'inertisation. On ajoute 220 ml de billes de verre d'un diamètre de 2 mm et on chauffe le récipient à 65 °C à l'aide du bain thermostaté. On broie ensuite le mélange pendant 5 min à une température de 65 à 75 °C, puis on vidange le récipient. On sépare les billes du mélange par filtration sur une grille d'un diamètre de 1 mm, on récupère le mélange dans une cartouche filtrante et on prélève un échantillon pour la mesure de la taille des particules. On sépare l'huile de la biomasse par pressage. On répète la procédure une deuxième fois.

On recueille 225 g d'huile et 75 g de tourteau. On filtre l'huile pressée à 50 °C, puis on la désodorise à 180 °C, 1 mbar pendant 2,2 h. On obtient finalement 210 g d'une huile limpide d'odeur neutre et de coloration jaune-claire. On détermine la teneur en ARA de l'huile par l'analyse GC et on calcule le taux d'incorporation de l'ARA.

### Exemple 13- Préparation par broyage avec de l'huile MCT dans un broyeur à billes.

On répète l'essai décrit dans l'exemple 12 en utilisant de l'huile MCT en remplacement de l'oléine de palme. On recueille finalement 235 g d'huile et 65 g de tourteau.

On filtre l'huile pressée à 50 °C, puis on la désodorise à 180 °C, 1 mbar pendant 2,3 h. On obtient finalement 220 g d'une huile limpide d'odeur neutre et de coloration jaune-claire. On détermine la teneur en ARA de l'huile par l'analyse GC et on calcule le taux d'incorporation de l'ARA.

### Exemple 14- Préparation par broyage avec de l'huile de tournesol riche en acide oléique dans un broyeur colloïdal.

### Equipement:

Broyeur colloïdal FRYMA MZ 80.
Centrifugeuse à panier PADBERG.
Filtre-cloche thermostaté MAVAG 300 ml.
Désodoriseur de laboratoire selon J.HEIDE-JENSEN (JAOCS; Vol. 40; 223-224; 1963) avec ballon de 1000 ml.

### Procédure:

On introduit 2800 g d'huile de tournesol riche en acide oléique et 1200 g de biomasse dans le récipient du broyeur. Le broyage s'effectue par recirculation du mélange dans le broyeur sous atmosphère inerte pendant 10 minutes à une température de 40 à 70 °C. On récupère le mélange et on prélève un échantillon pour la mesure de la taille des particules. On sépare l'huile de la biomasse à l'aide de la centrifugeuse à panier. On recueille finalement 2400 g d'huile et 1400 g de tourteau.

On filtre 200 g de l'huile centrifugée à 50°C, puis on la désodorise à 180°C, 1 millibar pendant 2 heures. On obtient finalement 190 g d'une huile limpide d'odeur neutre et de coloration jaune-claire. On détermine la teneur en ARA de l'huile par l'analyse GC et on calcule le taux d'incorporation de l'ARA. Les résultats sont indiqués dans le tableau 3 ci-après:

**Tableau 3**

| Exemple | Taille des particules (Malvern Mastersizer) micron D (v, 0,9)* | g ARA dans 100 g huile | % taux d'incorporation de l'ARA |
|---|---|---|---|
| 11 | 3000 | 3,5 | 66,1 |
| 12 | 75 | 5,0 | 94,3 |
| 13 | 60 | 5,0 | 94,3 |
| 14 | 115 | 5,25 | 99,0 |

| | | | |
|---|---|---|---|
| *Micron, D (v, 0,9): signifie que 90 % en volume des particules ont un diamètre inférieur à D. | | | |

### Exemple 15: Incorporation de DHA dans l'huile de tournesol riche en acide oléique

### Equipement:

Broyeur colloïdal FRYMA MZ 80.
Centrifugeuse à panier PADBERG.
Filtre-cloche thermostaté MAVAG 300 ml.
Désodoriseur de laboratoire selon J.HEIDE-JENSEN (JAOCS; Vol. 40; 223-224; 1963) avec ballon de 1000 ml.

### Procédure:

On répète la procédure de l'exemple 14 en traitant 1200 g d'une biomasse contenant 25 % d'une huile avec une teneur en DHA de 40 %. On recueille 2500 g d'huile avec une teneur en DHA de 3,5 % que l'on désodorise.

### Exemples 16-21

16-17. On prépare une formule infantile pour prématurés enrichie en ARA à partir de l'huile préparée par le procédé des exemples 12 ou 13 et on y ajoute d'autres huiles par exemple dans les proportions indiquées dans le tableau 4 ci-dessous, des protéines, le cas échéant hydrolysées, des hydrates de carbone et le cas échéant des vitamines et des oligoéléments.

**Tableau 4**

| | Exemple 16 | Exemple 17 |
|---|---|---|
| Huile de l'exemple 12 | 4 | - |
| Huile de l'exemple 13 | - | 4 |
| Huile de poisson | 1,5 | 1,5 |
| Huile MCT | 27 | 25 |
| Huile de soja | 23 | 23 |
| Oléine de palme | 44,5 | 35 |
| Huile de tournesol riche en acide oléique | - | 11,5 |
| Total | 100 | 100 |

18-19. On prépare une formule infantile pour nourrissons nés à terme enrichie en ARA à partir de l'huile support préparée par le procédé des exemples 13 ou 14 et on y ajoute d'autres huiles par exemple dans les proportions indiquées dans le tableau 5 ci-dessous, des protéines, le cas échéant hydrolysées, des hydrates de carbone et le cas échéant des vitamines et des oligoéléments.

**Tableau 5**

| | Exemple 18 | Exemple 19 |
|---|---|---|
| Huile de l'exemple 13 | 4,5 | - |
| Huile de l'exemple 14 | - | 7 |
| Huile de poisson | 1,5 | 1,5 |
| Huile de coco | 20 | 27,5 |
| Huile de soja | 20 | 20 |
| Oléine de palme | 54 | 44 |
| Total | 100 | 100 |

20-21. On prépare un lait de suite pour petits enfants enrichi en ARA à partir de l'huile support préparée par le procédé de 1' exemple 12 ou enrichi en DHA à partir de l'huile support préparée par le procédé de l'exemple 15, et on y ajoute d'autres huiles dans les proportions indiquées dans le tableau 6 ci-dessous, des protéines, le cas échéant hydrolysées, des hydrates de carbone et le cas échéant des vitamines et des oligoéléments.

**Tableau 6**

| | Exemple 20 | Exemple 21 |
|---|---|---|
| Huile de l'exemple 12 | 4 | - |
| Huile de l'exemple 15 | - | 7 |
| Huile de poisson | 1,5 | - |
| Huile de palmiste | 27 | - |
| Huile de coco | - | 19 |
| Huile de soja | 23 | - |
| Huile de colza | - | 30 |
| Oléine de palme | 44,5 | 44 |
| Total | 100 | 100 |

### Exemple 22

On prépare un lait liquide enrichi en DHA à raison de 1% de DHA dans la phase grasse de la manière suivante:
On pasteurise séparément un lait entier contenant 3,92 % de matière grasse et
8,58 % de solides non gras et un lait maigre contenant 0,05 % de matière grasse et 9 % de solides non gras, en les traitant à 87° C pendant 12 s.

On mélange ensuite 34,69 kg de lait entier et 160,26 kg de lait maigre, refroidi à 15° C, puis on incorpore à ce mélange un prémélange de 1,08 kg d'huile obtenue selon l'exemple 15 (huile de tournesol à haute teneur en acide oléique, contenant 3,5 % de DHA), 1,08 kg d'huile de soja et 1 g de vitamine E porté à 50° C au moyen d'un moulin colloïdal.

### Produit stérilisé:

Après réchauffage à 80° C dans un échangeur à plaques, on stérilise le liquide par UHT à 148° C pendant 5 s. Après refroidissement à 78° C, on l'homogénéise en deux étages, à 200 bar, puis à 50 bar, on le refroidit à 20° C et on le conditionne aseptiquement dans des emballages de type brique ayant été préalablement stérilisés, les étapes d'homogénéisation, de refroidissement et de remplissage ayant lieu de manière aseptique.

### Produit pasteurisé:

On chauffe le liquide à 72° C pendant 15 s dans un échangeur à plaques, on l'homogénéise en deux étages à 200 bar, puis à 50 bar, on le refroidit à 4° C et on le conditionne dans des emballage de type brique.

### Exemple 23

A titre de supplément nutritionnel, on encapsule une huile préparée selon l'exemple 12, 13 ou 14 contenant l'ARA ou une huile préparée selon l'exemple 15, contenant le DHA à raison de 500 mg d'huile dans des gélules.

## Revendications

1. Huile stable entrant dans la composition d'un produit alimentaire, nutritionnel, pharmaceutique ou cosmétique destiné à l'usage humain ou animal, contenant un ou plusieurs acides gras polyinsaturés à longue chaîne provenant d'une biomasse, sous forme de triacylglycérols servant à la fois de milieu de transfert des dits acides gras polyinsaturés à longue chaîne à partir de la biomasse et de support des dits acides gras polyinsaturés à longue chaîne dans le dit produit alimentaire, nutritionnel, pharmaceutique ou cosmétique et dans laquelle les acides gras polyinsaturés à longue chaîne sont incorporés de telle manière que, au moins 60 % en poids des acides gras polyinsaturés à longue chaîne présent dans la biomasse se retrouve dans l'huile et que moins de 10 % du phosphore présent dans la biomasse se retrouve dans l'huile et que l'huile ne contient pas plus de 10% en poids d'acides gras polyinsaturés à longue chaîne.

2. Huile selon la revendication 1, dans laquelle les acides gras polyinsaturés à longue chaîne sont choisis parmi l'acide arachidonique, l'acide dihomogammalinolénique, l'acide eicosapentaenoique ou l'acide docosahexaénoique.

3. Huile selon la revendication 2, dans laquelle l'acide gras polyinsaturé à longue chaîne est l'acide arachidonique.

4. Huile selon la revendication 2, dans laquelle l'acide gras polyinsaturé à longue chaîne est l'acide docosahexaénoique.

5. Procédé de préparation d'une huile stable entrant dans la composition d'un produit alimentaire, nutritionnel, pharmaceutique ou cosmétique destiné à l'usage humain ou animal, contenant un ou plusieurs acides gras polyinsaturés à longue chaîne provenant d'une biomasse, dans lequel on met en contact une huile support servant à la fois de milieu de transfert des dits acides gras polyinsaturés à longue chaîne à partir de la biomasse et de support des dits acides gras polyinsaturés à longue chaîne dans le dit produit alimentaire, nutritionnel, pharmaceutique ou cosmétique avec une biomasse sèche provenant de la culture d'un microorganisme contenant le dit un ou des acides gras polyinsaturés à longue chaîne, de manière à transférer le ou les acides gras polyinsaturés à longue chaîne sous forme de triacylglycérols vers le dit support de telle manière que, au moins 60 % en poids des acides gras polyinsaturés à longue chaîne présent dans la biomasse se retrouve dans l'huile et que moins de 10 % du phosphore présent dans la biomasse se retrouve dans l'huile, on sépare l'huile contenant le ou les dits acides gras du résidu de biomasse par pressage et filtration et on la désodorise dans des conditions ménageantes.

6. Procédé selon la revendication 5, dans lequel on réduit les dimensions des particules de biomasse sèche pour briser les parois des cellules de microorganismes et augmenter ainsi la surface de contact entre l'huile et la biomasse, en la broyant avant de la mélanger avec l'huile support.

7. Procédé selon la revendication 5, dans lequel on traite la biomasse sous forte pression en présence de l'huile support, puis on sépare l'huile obtenue contenant l'acide gras polyinsaturé à longue chaîne d'avec la biomasse par pressage et filtration.

8. Procédé selon la revendication 5, dans lequel on soumet la biomasse à une mouture en présence de l'huile support dans des conditions douces, à température modérée sous atmosphère inerte, notamment sous couche d'azote.

9. Procédé selon l'une des revendications 5 à 8, dans lequel on effectue une filtration de finition de manière à éliminer les particules fines de biomasse.

## Claims

1. Stable oil used in the composition of a food, nutritional, pharmaceutical or cosmetic product intended for human or animal use, containing one or more long-chain polyunsaturated fatty acids originating from a biomass, in the form of triacylglycerols, serving both as a transfer medium for said long-chain polyunsaturated fatty acids originating from the biomass and as a carrier for said long-chain polyunsaturated fatty acids in said food, nutritional, pharmaceutical or cosmetic product, and wherein the long-chain polyunsaturated fatty acids are incorporated in such a way that at least 60 percent by weight of the long-chain polyunsaturated fatty acids present in the biomass are contained in the oil and less than 10% of the phosphorus present in the biomass is contained in the oil and that the oil contains no more than 10 percent by weight of long-chain polyunsaturated fatty acids.

2. Oil according to claim 1, wherein the long-chain polyunsaturated fatty acids are chosen from arachidonic acid, dihomo-gamma-linolenic acid, eicosapentaenoic acid or docosahexaenoic acid.

3. Oil according to claim 2, wherein the long-chain polyunsaturated fatty acid is arachidonic acid.

4. Oil according to claim 2, wherein the long-chain polyunsaturated fatty acid is docosahexaenoic acid.

5. Process for preparing a stable oil used in the composition of a food, nutritional, pharmaceutical or cosmetic product intended for human or animal use, containing one or more long-chain polyunsaturated fatty acids originating from a biomass, wherein a carrier oil serving both as a transfer medium for said long-chain polyunsaturated fatty acids originating from the biomass and as a carrier for said long-chain polyunsaturated fatty acids in said food, nutritional, pharmaceutical or cosmetic product is brought into contact with a dry biomass originating from the culture of a microorganism containing said long-chain polyunsaturated fatty acid(s) so as to transfer the long-chain polyunsaturated fatty acid(s) in the form of triacylglycerols to said carrier, in such a way that at least 60 percent by weight of the long-chain polyunsaturated fatty acids present in the biomass are contained in the oil and less than 10% of the phosphorus present in the biomass is contained in the oil, the oil containing the fatty acid(s) is separated from the biomass residue by pressing and filtration and is deodorised under controlled conditions.

6. Process according to claim 5, wherein the dimensions of the particles of dry biomass are reduced to break down the walls of the microorganism cells and thereby increase the surface area of contact between the oil and the biomass, by grinding it prior to mixing it with the carrier oil.

7. Process according to claim 5, wherein the biomass is processed under high pressure in the presence of the carrier oil, then the oil obtained containing the long-chain polyunsaturated fatty acid is separated from the biomass by pressing and filtration.

8. Process according to claim 5, wherein the biomass is ground in the presence of the carrier oil under gentle conditions, at a moderate temperature and under an inert atmosphere, particularly under a nitrogen blanket.

9. Process according to one of claims 5 to 8, wherein a final filtration is performed in order to eliminate the fine particles of biomass.

## Patentansprüche

1. Stabiles Öl als Bestandteil einer Zusammensetzung eines Lebensmittelsprodukts, Ernährungsprodukts, eines pharmazeutischen oder kosmetischen Produkts für Menschen oder Tiere, das ein oder mehrere langkettige mehrfach ungesättigte Fettsäuren aus Biomasse in Form von Triacylglycerinen enthält und gleichzeitig als Medium zur Übertragung der langkettigen mehrfach ungesättigten Fettsäuren aus der Biomasse und als Träger dieser langkettigen mehrfach ungesättigten Fettsäuren in dem Lebensmittelprodukt, Ernährungsprodukt, pharmazeutischen oder kosmetischen Produkt dient und bei dem die langkettigen mehrfach ungesättigten Fettsäuren so eingeführt werden, dass mindestens 60 Gew.-% der in der Biomasse vorhandenen langkettigen mehrfach ungesättigten Fettsäuren in das Öl übergehen und dass weniger als 10 % des in der Biomasse vorhandenen Phosphors in das Öl übergehen und dass das Öl nicht mehr als 10 Gew.-% langkettige mehrfach ungesättigte Fettsäuren enthält.

2. Öl nach Anspruch 1, bei dem die langkettigen mehrfach ungesättigten Fettsäuren aus Arachidonsäure, Dihomogammalinolensäure, Eicosapentaensäure oder Docosahexaensäure ausgewählt sind.

3. Öl nach Anspruch 2, bei dem die langkettige mehrfach ungesättigte Fettsäure Arachidonsäure ist.

4. Öl nach Anspruch 2, bei dem die langkettige mehrfach ungesättigte Fettsäure Docosahexaensäure ist.

5. Verfahren zur Herstellung eines stabiles Öls als Bestandteil der Zusammensetzung eines Lebensmittelsprodukts, Ernährungsprodukts, eines pharmazeutischen oder kosmetischen Produkts für Menschen oder Tiere, das ein oder mehrere langkettige mehrfach ungesättigte Fettsäuren aus Biomasse enthält, bei dem man ein Trägeröl, das gleichzeitig als Medium für die Übertragung der langkettigen mehrfach ungesättigten Fettsäuren aus der Biomasse und als Träger der langkettigen mehrfach ungesättigten Fettsäuren in dem Lebensmittelprodukt, Ernährungsprodukt, pharmazeutischen oder kosmetischen Produkt dient, mit einer die langkettige mehrfach ungesättigte Fettsäure oder Fettsäuren enthaltenden trockenen Biomasse aus der Kultur eines Mikroorganismus so in Kontakt bringt, dass die langkettige mehrfach ungesättigte Fettsäure oder Fettsäuren in Form von Triacylglycerinen auf diesen Träger so übertragen werden, dass mindestens 60 Gew.-% der in der Biomasse vorhandenen langkettigen mehrfach ungesättigten Fettsäuren in das Öl übergehen und dass weniger als 10 % des in der Biomasse vorhandenen Phosphors in das Öl übergehen, man das die Fettsäure oder Fettsäuren enthaltende Öl von dem Biomasserückstand durch Pressen und Filtration trennt und es unter schonenden Bedingungen deodoriert.

6. Verfahren nach Anspruch 5, bei dem man die Abmessungen der trockenen Biomasseteilchen reduziert, um die Wände der Zellen der Mikroorganismen zu brechen und auf diese die Kontaktfläche zwischen Öl und Biomasse zu vergrößern, indem sie vor der Mischung mit dem Trägeröl zerkleinert wird.

7. Verfahren nach nach Anspruch 5, bei dem man die Biomasse in Gegenwart des Trägeröls unter hohem Druck behandelt und dann das erhaltene die langkettige mehrfach ungesättigte Fettsäure enthaltende Öl von der Biomasse durch Pressen und Filtration trennt.

8. Verfahren nach Anspruch 5, bei dem man die Biomasse einer Mahlung in Gegenwart des Trägeröls unter milden Bedingungen bei mäßiger Temperatur unter Inertatmosphäre, insbesondere unter einer Stickstoffschicht, unterzieht.

9. Verfahren nach einem der Ansprüche 5 bis 8, bei dem man eine Endfiltration so vornimmt, dass die feinen Biomasseteilchen entfernt werden.
